(19) Europäisches Patentamt
European Patent Office
Office européen des brevets

(11) **EP 3 754 624 A2**

(12) **EUROPEAN PATENT APPLICATION**

(43) Date of publication:
**23.12.2020 Bulletin 2020/52**

(51) Int Cl.:
**G08B 21/04** *(2006.01)*     **A61B 5/11** *(2006.01)*

(21) Application number: **20176721.7**

(22) Date of filing: **27.05.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.05.2019   KR 20190062535**

(71) Applicant: **Xandar Kardian**
**Seoul 04793 (KR)**

(72) Inventors:
• **Yang, Sun Jong**
  **Gangwon-do 26101 (KR)**
• **Choi, Jeong Woo**
  **Seoul 04715 (KR)**
• **Cho, Hyun Ah**
  **Seoul 08001 (KR)**
• **Yim, Dae Hyeon**
  **Seoul 04745 (KR)**

(74) Representative: **Charrier Rapp & Liebau**
**Patentanwälte PartG mbB**
**Fuggerstraße 20**
**86150 Augsburg (DE)**

(54) **APPARATUS FOR DETECTING FALL AND RISE**

(57)     Disclosed is an apparatus for detecting fall and rise. The apparatus includes a first sensor unit disposed at a first height and configured to sense a movement of a resident, a second sensor unit disposed at a second height higher than the first height and configured to sense the movement of the resident, and a controller configured to sense a falling behavior and a rising behavior of the resident based on distances from the resident measured by the first sensor unit and the second sensor unit. The controller sets the first sensor unit and the second sensor unit such that, among three axis coordinates of the first sensor unit and the second sensor unit in a 3D orthogonal coordinate system, two axis coordinates thereof coincide with each other.

FIG. 5a

EP 3 754 624 A2

## Description

## BACKGROUND

### (a) Technical Field

The present disclosure relates to an apparatus for detecting fall and rise which determines a falling behavior of a resident by sensing the movement of the resident.

### (b) Background Art

As interest in health care is increasing, interest in services for assisting elderly persons or disabled persons, who have mobility difficulties, to perform their everyday lives in safety is increasing. Since it is difficult for elderly persons or disabled persons, who live alone at home, to stay with guardians at all times, research on a service to sense the sudden fall of an elderly person or a disabled person is underway. Fall in which a person suddenly collapses is a phenomenon in which the person standing perpendicular to the ground collapses into a state in which the person is parallel to the ground and is thus injured. Such fall may be more injurious to elderly persons or disabled persons, whose physical functions are deteriorated.

Conventionally, a sensor is attached to a resident as technology to sense fall, but in this case, erroneous recognition by the sensor due to the movement of the resident frequently occurs and the resident must always wear the sensor, which is inconvenient. Further, in various cases, i.e., during a process of attaching the sensor to the resident, in the case in which a battery of the sensor is discharged, etc., the sensor may not sense whether or not the resident has fallen.

The above information disclosed in this Background section is only for enhancement of understanding of the background of the invention and therefore it may contain information that does not form the prior art that is already known in this country to a person of ordinary skill in the art.

## SUMMARY OF THE DISCLOSURE

The present invention has been made in an effort to solve the above-described problems associated with the prior art, and it is an object of the present invention to provide an apparatus for detecting fall and rise which may sense falling and rising behaviors of a resident by sensing the height of the resident in real time.

It is another object of the present invention to provide an apparatus for detecting fall and rise which may detect whether or not a resident has left a bed by sensing the height of the resident in real time.

In one aspect, the present invention provides an apparatus for detecting fall and rise, including a first sensor unit disposed at a first height and configured to sense a movement of a resident, a second sensor unit disposed at a second height higher than the first height and configured to sense the movement of the resident, and a controller configured to sense a falling behavior and a rising behavior of the resident based on distances from the resident measured by the first sensor unit and the second sensor unit, wherein the controller sets the first sensor unit and the second sensor unit such that, among three axis coordinates of the first sensor unit and the second sensor unit in a 3D orthogonal coordinate system, two axis coordinates thereof coincide with each other.

In a preferred embodiment, the controller may calculate a height of the resident based on the distances from the resident measured by the first sensor unit and the second sensor unit, and determine the falling behavior and the rising behavior of the resident using the height of the resident.

In another preferred embodiment, the first sensor unit and the second sensor unit may measure shortest distances from the resident, and the controller may calculates the height of the resident based on the shortest distances, the coordinates of the first sensor unit, and the coordinates of the second sensor unit.

In still another preferred embodiment, when the rising behavior of the resident is not sensed within a designated time after sensing the falling behavior of the resident, the controller may determine that the resident is in danger.

In yet another preferred embodiment, the first sensor unit and the second sensor unit may sense biometric signals of the resident at specific positions of the resident, and the specific position may mean a position of a body part of the resident at which a shortest distance from each of the first sensor unit and the second sensor unit to the resident is derived.

In still yet another preferred embodiment, when specific positions of the resident sensed by the first sensor unit and the second sensor unit are different, the controller may calculate a height of the resident assuming that a first specific position sensed by the first sensor unit and a second specific position sensed by the second sensor unit are the same.

In a further preferred embodiment, when a biometric signal is not sensed from a specific position of the resident sensed by each of the first sensor unit and the second sensor unit, the controller may control each of the first sensor unit and the second sensor unit so as to change the specific position of the resident sensed by each of the first sensor unit and the second sensor unit.

In another further preferred embodiment, when a biometric signal is sensed from the changed specific position, the controller may determine the falling behavior and the rising behavior by calculating a height of the resident based on the changed specific position.

In still another further preferred embodiment, when a bed is disposed in a residence place of the resident, the controller may pre-store information about a distance between an axis on which the first sensor unit and the second sensor unit are disposed, the bed and a height of the bed.

In yet another further preferred embodiment, the controller may not determine a change in the movement of the resident within a designated range from the distance between the axis and the bed as the falling behavior based on the information about the distance between the axis and the bed.

In still yet another further preferred embodiment, when a position of the resident sensed by the first sensor unit and the second sensor unit is within an error range from the height of the bed, the controller may determine that the resident is located on the bed and is thus not in danger.

In a still further preferred embodiment, the controller may set a rising height of the resident based on the height of the bed, and the controller may determine that the resident has risen when a position of the resident is higher than the rising height.

In a yet still further preferred embodiment, the controller may determine whether or not a position of the resident deviates from the bed beyond an error range using the height of the bed and the distance between the axis and the bed.

In still another preferred embodiment, the apparatus may further include a communication unit configured to inform an outside of position information of the resident, and, upon determining that the position of the resident deviates from the bed beyond the error range, the communication unit may output a signal indicating that the resident has left the bed.

In yet another preferred embodiment, the first sensor unit may be disposed on a bed, on which the resident is located, the first sensor unit may have a sensing region to determine whether or not the resident has left the bed, and the controller may determine that the resident is not in danger, when the resident is present within the sensing region.

In still yet another preferred embodiment, the apparatus may further include a database configured to store behavior pattern information about human movements, and the controller may determine a state of the resident by comparing a pattern of the movement of the resident sensed by the first sensor unit and the second sensor unit with the behavior pattern information stored in the database.

In still another aspect, the present invention provides an apparatus for detecting fall and rise including a first sensor unit disposed at a first height and configured to sense a movement of a resident, a second sensor unit disposed at a second height higher than the first height and configured to sense the movement of the resident, and a controller configured to sense a falling behavior and a rising behavior of the resident based on distances from the resident measured by the first sensor unit and the second sensor unit, wherein the first sensor unit and the second sensor unit are disposed so as to overlap each other in a direction perpendicular to a floor of a residence place of the resident.

In a preferred embodiment, the controller may set an orthogonal coordinate system configured to define a direction from the first sensor unit to the second sensor unit as one axis, and the controller may set coordinates of the first sensor unit and coordinates of the second sensor unit such that other coordinates of the first and the second sensor units are the same except for the coordinates of the first and second sensor units on the one axis.

In another preferred embodiment, the first sensor unit and the second sensor unit may measure respective shortest distances from the resident, and the controller may sense the falling behavior and the rising behavior of the resident by calculating a height of the resident based on the shortest distances and a distance between the first sensor unit and the second sensor unit.

In still another preferred embodiment, the controller may calculate a distance between the resident and the one axis based on the shortest distances, the coordinates of the first sensor unit, the coordinates of the second sensor unit and a height of the resident.

Other aspects and preferred embodiments of the invention are discussed infra.

The above and other features of the invention are discussed infra.

## BRIEF DESCRIPTION OF THE DRAWINGS

The above and other features of the present invention will now be described in detail with reference to certain exemplary embodiments thereof illustrated in the accompanying drawings which are given hereinbelow by way of illustration only, and thus are not limitative of the present invention, and wherein:

FIG. 1 is a view illustrating a position where an apparatus for detecting fall and rise according to one embodiment of the present invention is provided;
FIG. 2 is a block diagram of the apparatus for detecting fall and rise according to one embodiment of the present invention;
FIG. 3 is a block diagram of a database shown in FIG. 2;
FIG. 4 is a view illustrating the positions of a first sensor unit and a second sensor unit according to one embodiment of the present invention, placed in an orthogonal coordinate system;
FIGs. 5A and 5B are views illustrating detection of fall and rise of a resident by the apparatus for detecting fall and rise according to one embodiment of the present invention;
FIG. 6 is a view illustrating sensing of biometric signals at specific positions of a resident according to one embodiment of the present invention;
FIG. 7 is a view illustrating sensing as to whether or not a resident has left a bed according to one embodiment of the present invention; and
FIG. 8 is a view illustrating sensing as to whether or not a resident is lying on a bed according to one embodiment of the present invention.

It should be understood that the appended drawings are not necessarily to scale, presenting a somewhat simplified representation of various preferred features illustrative of the basic principles of the invention. The specific design features of the present invention as disclosed herein, including, for example, specific dimensions, orientations, locations, and shapes, will be determined in part by the particular intended application and use environment.

In the figures, reference numbers refer to the same or equivalent parts of the present invention throughout the several figures of the drawings.

## DETAILED DESCRIPTION

Advantages and features of the present invention and methods for achieving them will become apparent from the descriptions of aspects herein below with reference to the accompanying drawings. However, the present invention is not limited to the aspects disclosed herein but may be implemented in various different forms. The aspects are provided to make the description of the present invention thorough and to fully convey the scope of the present invention to those skilled in the art. It is to be noted that the scope of the present invention is defined only by the claims. In the following description of the embodiments, the same or similar elements are denoted by the same reference numerals even though they are depicted in different drawings.

In the following description of the embodiments, terms, such as "... unit", "... part", "...module", etc., mean units to process at least one function or operation, and these may be implemented by hardware, software or a combination of hardware and software.

In addition, in the following description of the embodiments, terms, such as "first", "second", etc., may be used only to distinguish one element from other elements, and do not limit the sequence of the elements.

Hereinafter reference will now be made in detail to various embodiments of the present invention, examples of which are illustrated in the accompanying drawings and described below. While the invention will be described in conjunction with exemplary embodiments, it will be understood that present description is not intended to limit the invention to those exemplary embodiments. On the contrary, the invention is intended to cover not only the exemplary embodiments, but also various alternatives, modifications, equivalents and other embodiments, which may be included within the spirit and scope of the invention as defined by the appended claims.

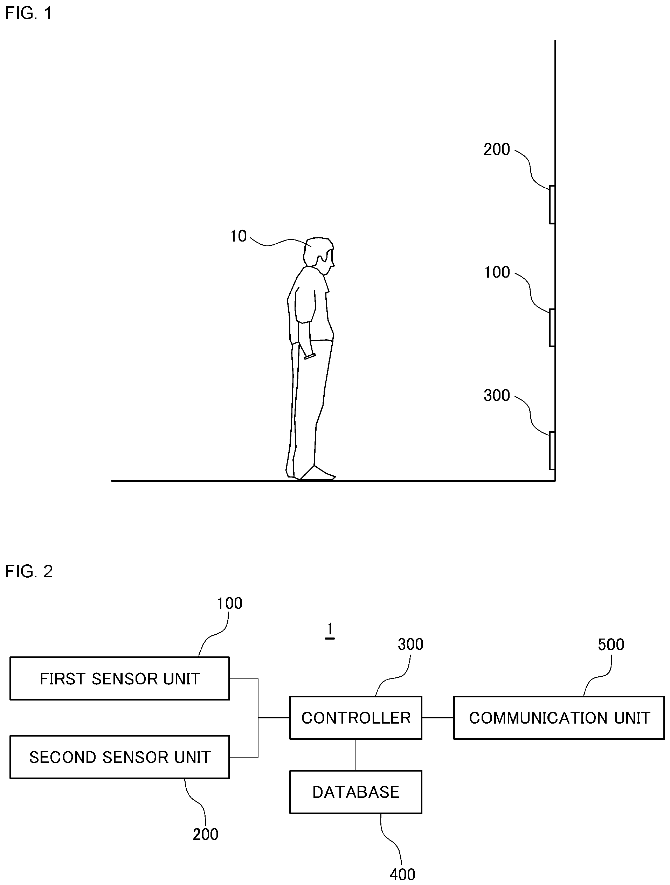

FIG. 1 is a view illustrating a position where an apparatus for detecting fall and rise according to one embodiment of the present invention is provided, and FIG. 2 is a block diagram of the apparatus for detecting fall and rise according to one embodiment of the present invention.

Referring to FIGs. 1 and 2, an apparatus 1 for detecting fall and rise may include a first sensor unit 100, a second sensor unit 200, a controller 300, a database 400 and a communication unit 500. The apparatus 1 for detecting fall and rise may be disposed in an indoor space, in which a resident 10 is located, and detect the falling and rising behaviors of the resident 10. In the apparatus 1 for detecting fall and rise, the first sensor unit 100, the controller 300, the database 400 and the communication unit 500 may be configured as one module, and the second sensor unit 200, the controller 300, the database 400 and the communication unit 500 may be configured as another module. That is, the controller 300, the database 400 and the communication unit 500 may be modularized together with the first sensor unit 100 or the second sensor unit 200. However, the present invention is not limited thereto, and the database 400 may be separately provided.

The first sensor unit 100 may be disposed at a first height and sense the movement of the resident 10, and the second sensor unit 200 may be disposed at a second height and sense the movement of the resident 10. The second height may be higher than the first height. For example, the first height may be 1m, and the second height may be 2m. However, the first height and the second height are not limited to specific heights. The first sensor unit 100 and the second sensor unit 200 may be disposed in a direction perpendicular to the floor of the indoor space in which the resident 10 resides, and the first sensor unit 100 and the second sensor unit 200 may be disposed so as to overlap each other in the direction perpendicular to the floor of the indoor space. As one example, the first sensor unit 100 and the second sensor unit 200 may be adhered to a wall located in the indoor space. As another example, the first sensor unit 100 and the second sensor unit 200 may be provided in the form of a stand which is adhered to one pillar.

Each of the first sensor unit 100 and the second sensor unit 200 may be one of an impulse-radio ultra-wideband (IR-UWB) sensor, a light detection and ranging (LIDAR) device, a frequency-modulated continuous-wave radio detection and ranging (FMCW RADAR) device, and a Doppler RADAR device. Particularly, each of the first sensor unit 100 and the second sensor unit 200 may be an IR-UWB sensor. UWB is a radio technology which uses a frequency band of 500 MHz or more or has a value of 25% or more, defined as a fractional bandwidth. The fractional bandwidth means a signal bandwidth to a center frequency. UWB is a radio technology which uses a wideband frequency, and has various advantages, such as high range resolution, transmittance, robustness to narrowband noise, and compatibility with other devices sharing frequencies therewith. For example, UWB has resolution of a high precision range of 1 cm or less, and may thus detect the fine movement of a target object.

An impulse-radio ultra-wideband RADAR (hereinafter, referred to as UWB RADAR) system, in which such UWB is combined with RADAR, means a RADAR technology which recognizes the surrounding circumstances by transmitting an impulse signal having wideband characteristics in a frequency domain with a very short time duration and then receiving a signal reflected by an object and/or a person. In the UWB RADAR system, a signal generator generates an impulse signal having a time width of several nano-seconds to several pico-seconds and radiates the impulse signal at a wide angle or a narrowband angle through a transmission antenna. The radiated signal may be reflected by various objects or persons, and the reflected signal may be converted into a digital signal through a reception antenna and an analog-to-digital converter (ADC).

The first sensor unit 100 and the second sensor unit 200 may sense the respective shortest distances to the resident 10. The first sensor unit 100 and the second sensor unit 200 may sense the shortest distances to the resident 10 based on specific positions of the resident 10. For example, the specific position may mean one of body parts of the resident, such as the head, the torso or the leg of the resident 10, and the specific position may mean the position of a body part of the resident 10 at which the shortest distance from the first sensor unit 100 or the second sensor unit 200 to the resident is derived. Therefore, the specific position may mean the position of a body part of the resident 10 which is changed in real time depending on the movement of the resident 10. A first shortest distance between the first sensor unit 100 and the resident and a second shortest distance between the second sensor unit 200 may be the same or different. A first specific position sensed by the first sensor unit 100 and a second specific position sensed by the second sensor unit 200 may be the same or different. For example, when the first specific position and the second specific position are different, the controller 300 may assume that the first specific position and the second specific position are the same. The optimum information to determine rising and falling behaviors may be the height of a specific position of the resident 10. For example, if the resident 10 rises, the first sensor unit 100 and the second sensor unit 200 may sense the torso of the resident 10. If the resident 10 rises, the distances from the torso of the resident to the first sensor unit 100 and the second sensor unit 200 may be the shortest distances. On the other hand, if the resident 10 falls, various body parts of the resident 10, such as the torso, the head or the leg of the resident 10, may be defined as specific positions depending on the falling direction of the resident 10.

The first sensor unit 100 and the second sensor unit 200 may sense biometric information of the resident 10 generated from a specific position of the resident 10. The biometric information may include at least one of heart rate, movement or respiration of the resident 10.

The first sensor unit 100 and the second sensor unit 200 may sense movement of the breast or abdomen of the resident 10 by receiving a signal reflected by the resident 10, and thereby sense the heart rate or respiration of the resident 10. Further, the first sensor unit 100 and the second sensor unit 200 may sense the movement of the resident 10 by receiving a signal reflected in real time. Further, the first sensor unit 100 and the second sensor unit 200 may sense the size and shape of the resident 10 by receiving the signal reflected in real time. Therefore, the first sensor unit 100 and the second sensor unit 200 may determine a current state of the resident 10 by measuring biometric information of the resident 10, such as the heart rate, movement or respiration of the resident 10.

The controller 300 may sense the falling behavior and the rising behavior of the resident 10 based on the distances from the resident measured by the first sensor unit 100 and the second sensor unit 200. In more detail, the controller 300 may calculate the height of the resident 10 based on the first shortest distance corresponding to the distance from the first sensor unit 100 to the first specific position of the resident 10 and the second shortest distance corresponding to the distance from the second sensor unit 100 to the second specific position of the resident 10. Here, the height of the resident may mean the height of the first specific position or the second specific position and, in general, the first specific position and the second specific position may mean the same position. The controller 300 may set an orthogonal coordinate system in which a direction from the first sensor unit 100 to the second sensor unit 200 is defined as one axis. The controller 300 may set coordinates of the first sensor unit 100 and coordinates of the second sensor unit 200 such that two other coordinates of the first and the second sensor units 100 and 200 are the same except for the coordinates of the first and second sensor units 100 and 200 on this axis. That is, the first sensor unit 100 and the second sensor unit 200 may be set to be disposed at the same coordinates except for respective coordinates of the first and second sensor units 100 and 200 on one axis. Therefore, the controller 300 may calculate the height of the resident 10 by combining information about the first shortest distance and the second shortest distance with information about the distance between the first sensor unit 100 and the second sensor unit 200.

The controller 300 may determine the falling behavior and the rising behavior of the resident 10 based on the height of the resident 10. That is, the controller 300 may determine whether or not the resident 10 has fallen or risen based on a change in the height of the resident 10. For example, the controller 300 may determine that the resident 10 has risen when the height of the resident 10 is suddenly increased, and determine that the resident 10 has fallen when the height of the resident 10 is suddenly decreased.

The controller 300 may determine whether or not the resident 10 is in danger based on whether or not the rising behavior of the resident 10 occurs within a designated time after sensing the falling behavior of the resident 10. The controller 300 may determine the state of the resident 10 based on the information sensed by the first sensor unit 100 and the second sensor unit 200.

For example, if the first sensor unit 100 and the second sensor unit 300 sense the rising behavior of the resident 10 within the designated time after sensing the falling behavior of the resident 10, the controller 300 may determine that the resident is not in danger. If the falling behavior of the resident 10 occurs but the resident has fallen over by mistake, the resident 10 may generally rise within the designated time. In this case, the resident is not in danger. Therefore, the first sensor unit 100 and the second sensor unit 200 sense the movement of the resident 10 for the designated time after sensing the falling behavior of the resident 10, and the controller 300 determines that there is nothing wrong with the resident 10, when the resident 10 rises within the designated time. The designated time may mean a time which is predetermined by a designer. The controller 300 may store information about a first rising reference height to determine the rising behavior, and determine that the resident 10 has risen when the height of the resident 10 is higher than the first rising reference height. The first rising reference height may be defined as a value acquired by adding a distance corresponding to the length of the upper body of the resident 10 to a height at which the fall of the resident 10 is detected. For example, the height at which the fall of the resident 10 is detected may be about 20 cm, and the distance corresponding to the length of the upper body of the resident 10 may be 70 cm to 1 m. That is, the first rising reference height may be in the range from 90 cm to 1 m 20 cm. However, the first rising reference height may be freely changed by a designer.

For example, if the first sensor unit 100 and the second sensor unit 200 sense no rising behavior of the resident 10 within the designated time after sensing the falling behavior of the resident 10, the controller 300 may determine that the resident is in danger. If there is something wrong with the resident 10, the resident 10 may not rise within the designated time after the resident 10 has fallen. Therefore, if the first sensor unit 100 and the second sensor unit 200 sense no rising behavior of the resident 10 within the designated time after the resident 10 has fallen, the controller 300 may determine that the resident 10 is in danger.

The controller 300 may determine whether or not the resident 10 is in danger by detecting a pattern of the movement of the resident 10 after sensing the falling behavior of the resident 10. The controller 300 may determine whether or not the resident 10 is in danger by comparing the movement of the resident 10 with a movement pattern stored in the database 400.

The database 400 may store information necessary to analyze information sensed by the first sensor unit 100 and the second sensor unit 200 in the indoor space. For example, the database 400 may include information used to distinguish movements other than the movement of the resident 10, among movements which are capable of being sensed in the indoor space. The controller 300 may analyze the information sensed by the first sensor unit 100 and the second sensor unit 200 based on the information stored in the database 400.

The communication unit 500 may output a warning message, when the controller 300 determines that the resident 10 is in danger. As one example, the communication unit 500 may output a warning sound indicating that the resident 10 is in danger. As another example, the communication unit 500 may transmit the warning message to at least one terminal located outdoors using a wireless communication method. Here, the at least one terminal may be a terminal possessed by a guardian of the resident 10, or be terminals possessed by unspecified individuals who are located outdoors. If the at least one terminal is a terminal of a guardian of the resident, the guardian may recognize that there is something wrong with the resident 10. If the at least one terminal corresponds to terminals of unspecified individuals, the unspecified individuals may recognize that there is something wrong with the resident 10 living indoors and take appropriate measures. As yet another example, the communication unit 500 may transmit a warning message to a hospital and an emergency medical center using a wireless communication method. Here, the communication unit 500 may output information including a residential address of the resident together with the warning message. The wireless communication method may include a Bluetooth method, an RF communication method, a near field communication (NFC) method, or the like. Further, the wireless communication method may use signals output by the first sensor unit 100 and the second sensor unit 200 to sense the movement of the resident 10. That is, the wireless communication method may be a method for outputting signals through an impulse-radio ultra-wideband (IR-UWB) sensor, a light detection and ranging (LIDAR) device, a frequency-modulated continuous-wave (FMCW) RADAR device, or a Doppler RADAR device.

According to the embodiment of the present invention, the apparatus 1 for detecting fall and rise may increase reliability of determination of the falling behavior of the resident 10 by sensing whether or not both the falling behavior and the rising behavior of the resident 10 occur. The apparatus 1 for detecting fall and rise may more accurately determine whether or not the resident 10 has fallen due to a health problem by determining the falling behavior of the resident 10 based on whether or not the resident 10 rises within the designated time after the falling behavior of the resident 10.

Further, the apparatus 1 for detecting fall and rise sets the first sensor unit 100 and the second sensor unit 200 such that, among three axis coordinates of the first sensor unit 100 and the second sensor unit 200, two axis coordinates thereof coincide with each other, and may thus sense the rising and falling behaviors of the resident 10 using only information about the shortest distances from the first sensor unit 100 and the second sensor unit 200 to the resident 10 and information about the distance between the first sensor unit 100 and the second sensor unit 200.

Moreover, when the apparatus 1 for detecting fall and rise determines that the resident 10 has fallen, the apparatus 1 for detecting fall and rise automatically outputs a warning message, and thus, persons receiving the warning message may prepare for occurrence of a secondary accident involving the resident 10 having the health problem.

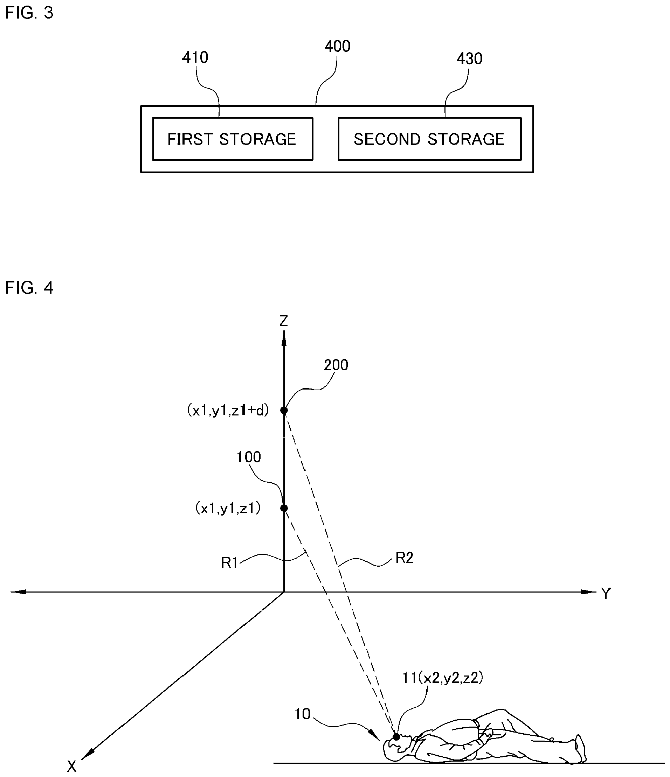

FIG. 3 is a block diagram of the database of FIG. 2.

Referring to FIGs. 2 and 3, the database 400 may include a first storage 410 and a second storage 430. The database 400 may store information used to accurately analyze the falling behavior of the resident 10 by the controller 300 based on various pieces of information acquired by the first sensor unit 100 and the second sensor unit 200.

The first storage 410 may store information about repetitive movements that may occur indoors. The repetitive movement may mean movement which is repeated on a designated cycle. For example, the repetitive movements may include the rotation of a fan, the movement of a second hand of a clock, etc. The controller 300 may determine that, among information about repetitive movements of objects sensed by the first sensor unit 100 and the second sensor unit 200, information which is the same as the information about repetitive movements stored in the database 400 is not information about the movement of the resident 10 or biometric information of the resident 10. That is, the controller 300 may analyze whether or not the resident 10 is in danger using only the movement of the resident 10 and the biometric information of the resident 10 out of various pieces of information measured by the first sensor unit 100 and the second sensor unit 200.

The second storage 430 may store first information about sizes, shapes, etc. of humans, second information about heart rates, respiration and movements of humans, and behavior pattern information about movements of humans, acquired through machine learning. The first sensor unit 100 and the second sensor unit 200 may sense the size of the resident 10 and the head part of the resident 10 based on the first information stored in the second storage 430. That is, the first sensor unit 100 and the second sensor unit 200 may determine which one of sensed signals corresponds to a signal regarding the head part of the resident 10, based on the first information. The controller 300 may detect whether or not the signals sensed by the first sensor unit 100 and the second sensor unit 200 are signals regarding a human heart rate, signals regarding a human respiration, or signals regarding a human movement through comparison with the second information. The controller 300 may determine the state of the resident 10 by comparing a pattern of the movement of the resident 10 sensed by the first sensor unit 100 and the second sensor unit 200 with the behavior pattern information. For example, the behavior pattern information may include various pieces of information, such as movement that a human conducts during exercise, a falling behavior in which a human suddenly falls, movement that a human takes after falling, etc. Therefore, the controller 300 may detect the meaning of the current movement performed by the resident 10 by matching the signals sensed by the first sensor unit 100 and the second sensor unit 200 with the behavior pattern information.

According to the embodiment of the present invention, the apparatus 1 for detecting fall and rise may accurately detect the state of the resident 10 using both the first sensor unit 100 and the second sensor unit 200, and accurately detect the meaning of the current movement performed by the resident 10 by comparing signals sensed by the first sensor unit 100 and the second sensor unit 200 with the information stored in the database 400.

FIG. 4 is a view illustrating the positions of the first sensor unit and the second sensor unit according to one embodiment of the present invention, placed in the orthogonal coordinate system. FIG. 4 illustrates a case in which the resident is lying on the floor after falling. FIG. 4 shows of the first sensor unit 100 and the second sensor unit 200 disposed in the 3D orthogonal coordinate system including the X-axis, the Y-axis and the Z-axis, and the X-axis, the Y-axis and the Z-axis may be orthogonal to one another.

Referring to FIGs. 2 to 4, the first sensor unit 100 may be disposed at the first height, and the coordinates of the first height may be (x1, y1, z1). The second sensor unit 200 may be disposed at the second height, and the coordinates of the second height may be (x1, y1, z1+d). The controller 300 may set the orthogonal coordinate system such that the X-axis and Y-axis coordinates of the first sensor unit 100 and the second sensor unit 200 coincide with each other. The first sensor unit 100 and the second sensor unit 200 overlap each other in a direction perpendicular to the floor of the indoor space in which the resident 10 resides, and thus, the controller 300 may set the orthogonal coordinate system such that a direction from the first sensor unit 100 to the second sensor unit 200 is defined as the Z-axis. Here, the distance between the first sensor unit 100 and the second sensor unit 200 may be defined as "d". For example, the distance d may be 1 m.

Each of the first sensor unit 100 and the second sensor unit 200 may measure the shortest distance of a specific position 11 of the resident 10. The coordinates of the specific position 11 may be (x2, y2, z2). In this embodiment, the head of the resident 10 may be defined as the specific position 11. Although a specific position sensed by the first sensor unit 100 and a specific position sensed by the second sensor unit 200 may be different, the controller 300 may calculate the height of the specific position 11 on the assumption that the specific position sensed by the first sensor unit 100 and the specific position sensed by the second sensor unit 200 are the same. The first shortest distance measured by the first sensor unit 100 may be defined as "R1", and the second shortest distance measured by the second sensor unit 200 may be defined as "R2". The controller 300 may calculate the height z2 of the resident 10 based on the first and second shortest distances R1 and R2 and the distance d between the first sensor unit 100 and the second sensor unit 200. Equations to calculate the height z2 of the resident 10 by the controller 300 are as follows.

$$R1^2 = (x2 - x1)^2 + (y2 - y1)^2 + (z2 - z1)^2$$

$$R2^2 = (x2 - x1)^2 + (y2 - y1)^2 + (z2 - z1 - d)^2$$

$$R2^2 - R1^2 = (z2 - z1 - d)^2 - (z2 - z1)^2$$

Here, z1 means the height at which the first sensor unit 100 is disposed, d means the distance between the first sensor unit 100 and the second sensor unit 200, and thus, z1 and d may be constant values. Further, R1 and R2 are values measured by the first sensor unit 100 and the second sensor unit 200, and thereby, the value of z2 may be calculated. That is, the controller 300 may calculate the height z2 of the resident.

The controller 300 may calculate a distance between the Z-axis and the resident 10 based on the height z2 of the resident 10, the first shortest distance, the second shortest distance and the coordinates of the first sensor unit 100 and the second sensor unit 200. The distance between the Z-axis and the resident 10 may be a parameter to check whether or not the resident 10 has left a bed. One function of determining whether or not the resident 10 has left the bed using the distance between the Z-axis and the resident 10 will be described below.

The controller 300 may determine the falling and rising behaviors of the resident 10 based on the height z2 of the resident 10. When the height z2 of the resident 10 is within a designated range from the floor, the controller 300 may determine that the resident 10 has fallen. When there is no rising behavior of the resident within the designated time after the resident 10 has fallen, the controller 300 may determine that the resident 10 is in danger.

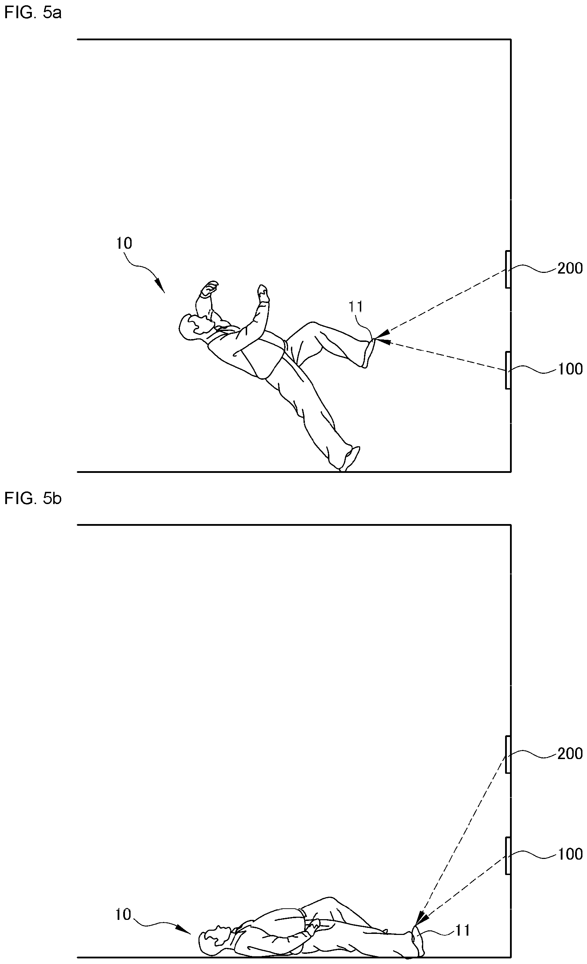

FIGs. 5A and 5B are views illustrating detection of fall and rise of the resident by the apparatus for detecting fall and rise according to one embodiment of the present invention. FIG. 5A is a view illustrating that, when the resident 10 has fallen, the apparatus 1 for detecting fall and rise senses the fall of the resident 10, and FIG. 5B is a view illustrating that the apparatus 1 for detecting fall and rise senses a lying state of the resident on the floor after falling.

Referring to FIGs. 2 and 5A, the first sensor unit 100 and the second sensor unit 200 may sense a change in the height of the resident 10 in real time. The first sensor unit 100 and the second sensor unit 200 may sense a change in the specific position 11 of the resident 10 in real time. The controller 300 may determine that the resident 10 is standing, when a difference between the height of the resident 10 and the floor is beyond the designated range. The controller 300 may accurately detect whether or not the resident 10 falls by matching the movement of the resident 10 sensed by the first sensor unit 100 and the second sensor unit 200 with the behavior pattern information stored in the database 400. Further, the controller 300 may detect whether or not the resident 10 has fallen by comparing the movement pattern of the resident 10 after falling with the behavior pattern information stored in the database 400.

Referring to FIGs. 2 and 5B, the controller 300 may determine that the resident 10 is lying on the floor after falling, when the difference between the height of the resident 10 and the floor is within the designated range. After the falling behavior of the resident 10 is sensed, the first sensor unit 100 and the second sensor unit 200 may sense the specific position 11 of the resident 10. The first sensor unit 100 and the second sensor unit 200 may measure the first shortest distance and the second shortest distance to the specific position 11 of the resident 10, and calculate the height of the resident 10 based on the first shortest distance and the second shortest distance. Here, the first sensor unit 100 and the second sensor unit 200 may sense a biometric signal generated from the specific position 11 of the resident 10. Even when the resident 10 has not fallen but an object has fallen on the floor, the controller 300 may determine that the resident 10 has fallen. However, because no biometric signal is generated from the object, the controller 300 may determine that the resident 10 has not fallen and the object has fallen.

The first sensor unit 100 and the second sensor unit 200 may measure the movement of the resident 10 falling down on the indoor floor, and the controller 300 may determine the state of the resident 10 based on information sensed by the first sensor unit 100 and the second sensor unit 200 and the behavior pattern information stored in the database 400. For example, the controller 300 may determine whether or not the resident 10 is intentionally lying on the floor or whether or not the resident 10 has fallen due to a health problem based on the behavior pattern information including various pieces of information, such as the movement which may be performed by the resident 10 after falling. In general, if the resident 10 has fallen due to a health problem, the resident 10 may not rapidly change his/her position and may not move at a high speed after falling. Since information about such movement of the resident 10 is stored in the database 400, the controller 300 may determine the state of the resident 10 after falling. If the controller 300 determines that the resident 10 is in danger, the controller 300 may control the communication unit 500 to output a warning message.

The controller 300 may determine whether or not the resident 10 is lying on the floor or has risen by calculating the height of the resident 10 in real time. If the resident 10 does not rise within the designated time after falling, the controller 300 may determine that the resident 10 is in danger. If the resident rises within the designated time after falling, the controller 300 may determine that the resident 10 is not in danger.

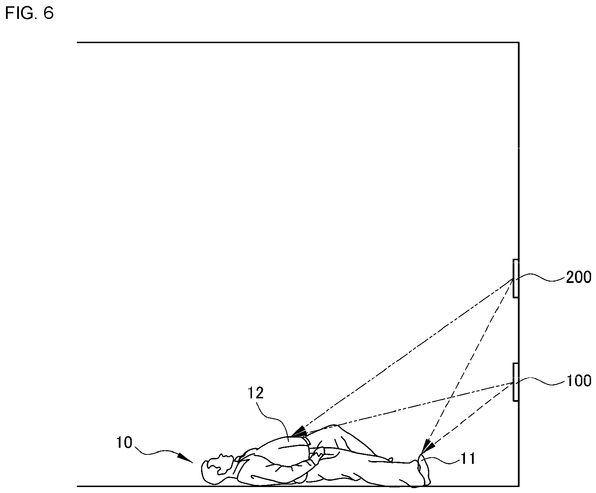

FIG. 6 is a view illustrating sensing of biometric signals at specific positions of the resident according to one embodiment of the present invention.

Referring to FIGs. 2 and 6, the first sensor unit 100 and the second sensor unit 200 may sense a specific position 11 of the resident 10 lying on the floor. The specific position 11 may be varied depending on the movement of the resident 10, and in this embodiment, the specific position 11 may mean the foot of the resident 10. The first sensor unit 100 and the second sensor unit 200 may sense a biometric signal generated from the specific position 11 of the resident 10. However, in this embodiment, the specific position 11 is the foot of the resident 10, and human feet do not generate a biometric signal. Therefore, the controller 300 may control the first sensor unit 100 and the second sensor unit 200 so as to change the specific position 11 of the resident 10. That is to say, the controller 300 may control the first sensor unit 100 and the second sensor unit 200 to sense other body parts than the foot of the resident 10. The controller 300 may change the sensing regions of the first sensor unit 100 and the second sensor unit 200 so as to sense a biometric signal generated from an area within a designated range from the existing specific position 11. For example, the controller 300 may search a new specific position 12, from which a biometric signal is sensed, while changing the sensing regions of the first sensor unit 100 and the second sensing unit 200 based on the existing specific position 11. For example, the new specific position 12 may be the torso of the resident 10, and the first sensor unit 100 and the second sensor unit 200 may sense a biometric signal including a heart rate or chest movement from the torso of the resident 10.

If the biometric signal is sensed from the changed specific position 12, i.e., the new specific position 12, the controller 300 may calculate the height of the resident 10 based on the new specific position 12. The controller 300 may determine the falling and rising behaviors of the resident 10 based on the newly calculated height of the resident 10.

According to this embodiment of the present invention, if no biometric signal is sensed from the specific position 11 of the resident 10, sensed by the first sensor unit 100 and the second sensor unit 200, the controller 300 may search the new specific position 12 by changing the sensing regions of the first sensor unit 100 and the second sensor unit 200. That is, the apparatus 1 for detecting fall and rise according to one embodiment of the present invention may detect the state of the resident 10 by sensing the biometric signal of the resident 10 in addition to detection of the falling and rising behaviors of the resident 10. Further, the apparatus 1 for detecting fall and rise may sense the specific position 11 so as to determine a change in the height of the resident 10, and change the sensing regions of the first and second sensing units 100 and 200 from the existing specific position 11 to the new specific position 12 so as to sense the biometric signal. Thereby, the apparatus 1 for detecting fall and rise may increase reliability in sensing of the biometric signal while sensing a change in the height of the resident 10.

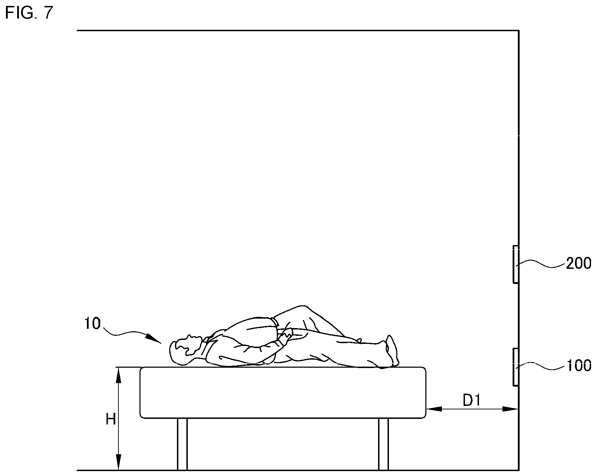

FIG. 7 is a view illustrating sensing as to whether or not a resident has left a bed according to one embodiment of the present invention.

Referring to FIGs. 2 and 7, a bed 50 may be disposed in the indoor space in which the resident 10 resides. If the resident 10 does not rise after lying on the bed 50, the apparatus 1 for detecting fall and rise may erroneously recognize that the resident 10 is in danger. Therefore, the controller 300 requires logic to recognize that the resident 10 is lying on the bed 50. For this purpose, the controller 300 may pre-store information about the distance D1 from the axis, on which the first sensor unit 100 and the second sensor unit 200 are disposed, to the bed 50 and the height H of the bed 50.

For example, the controller 300 may not determine a change in the movement of the resident 10, which is within a designated range from the distance D1, as a falling behavior based on the information about the distance D1. The resident 10 may lie on the bed 50 which is separated from the first sensor unit 100 and the second sensor unit 200 by the distance D1, and the controller 300 may not determine a behavior of the resident 10 lying on the bed 50 as a falling behavior.

For example, if the position of the resident 10 sensed by the first sensor unit 100 and the second sensor unit 200 is within the error range from the height H of the bed 50, the controller 300 may determine that the resident 10 is located on the bed 50. Here, the controller 300 may determine that the resident 10 is not in danger. In general, the upper surface of the bed 50 may have a height of 60 cm to 1 m from the floor. If the controller 300 determines that the position of the resident 10 sensed by the first sensor unit 100 and the second sensor unit 200 is within the predetermined error range from the height of the bed 50, the controller 300 may determine that the resident 10 has not fallen but is lying on the bed 50. Further, the controller 300 may determine whether or not the resident 10 is lying on the bed 50 based on the distance D1 between the bed 50 and the first and second sensor units 100 and 200.

For example, the controller 300 may determine whether or not the position of the resident 10 deviates from the bed 50 beyond an error range using the height H of the bed 50 and the distance D1. A patient with an advanced disease may be critical when he or she has left the bed 50. Therefore, the controller 300 may determine whether or not the resident 10 has left the bed 50. The communication unit 500 may inform the outside of position information of the resident 10. If the position of the resident 10 deviates from the bed 50 beyond the error range, the communication unit 500 may output a signal indicating that the resident 10 has left the bed 50. Such logic may be applied when the resident 10 is a patient with an advanced disease or when the apparatus 1 for detecting fall and rise is used in hospitals.

The controller 300 may set a second rising reference height based on the height of the bed 50. The second rising reference height may mean a height to determine whether or not the resident 10 lying on the bed 50 has risen. A change in the height of the resident 10 when the resident 10 lying on the floor has risen and a change in the height of the resident 10 when the resident 10 lying on the bed 50 has risen may be different. For example, when the resident 10 is lying on the floor, the controller 300 may determine that the height of the resident 10 is about 20 cm, and when the resident 10 is lying on the bed 50, the controller 300 may determine that the height of the resident 10 is similar to the height (60 cm to 1 m) of the bed 50. Therefore, the controller 300 may set the second rising reference height of the resident 10 upon determining that the resident 10 is lying on the bed 50 to be different from the first rising reference height of the resident 10 upon determining that the resident 10 is lying on the floor. For example, the second rising reference height of the resident 10 upon determining that the resident 10 is lying on the bed 50 may be defined as a value acquired by adding a distance corresponding to the length of the upper body of the resident 10 to the height H of the bed 50. For example, the height of the bed 50 may be 60 cm to 1 m, and the distance corresponding to the length of the upper body of the resident 10 may be 70 cm to 1 m. That is, the second rising reference height may be in the range from 1 m 30 cm to 2 m, which is greater than the first rising reference height. However, the second rising reference height may be freely changed by a designer.

The first sensor unit 100 and the second sensor unit 200 may sense a movement pattern of the resident 10 after the rising behavior of the resident 10, and the controller 300 may detect the state of the resident 10 by comparing the movement pattern of the resident with the information stored in the database 400. For example, the resident 10 may feel dizzy and thus stagger after rising and, and the controller 300 may determine that the resident 10 is in danger upon determining that a variance value of the movement of the resident 10 is a designated value or greater. Even when the resident 10 has left the bed 50, the resident 10 may fall after deviating from the sensing regions of the first sensor unit 100 and the second sensor unit 200. Therefore, the controller 300 may analyze the movement pattern of the resident 10, and control the communication unit 500 to generate an alarm upon determining that there is a risk of falling.

When the resident has left the bed 50 or the movement pattern of the resident 10 is unstable after the resident 10 has risen from the bed 50, the controller 300 may determine that the resident 10 is in danger. Here, the rising behavior of the resident 10 after lying on the bed 50 may be first sensed, and then, the controller 300 may determine whether or not the resident 10 has left the bed 50 and analyze the movement pattern of the resident 10. Although the apparatus 1 for detecting fall and rise may determine a behavior of the resident 10 lying on the bed 50 as a falling behavior, the apparatus 1 for detecting fall and rise may determine that the resident 10 lies on the bed 50 based on the height H of the bed 50 and the distance D1 between the bed 50 and the sensor units 100 and 200. Further, the apparatus 1 for detecting fall and rise may sense the rising behavior of the resident 10 after lying on the bed 50, and determine whether or not the resident 10 is in danger after rising.

According to this embodiment, the apparatus 1 for detecting fall and rise may include logic to prevent erroneous recognition of the resident 10 lying on the bed 50 disposed in the indoor space as falling. Further, the apparatus 1 for detecting fall and rise may detect a behavior of the resident 10 lying on the bed 50 and a behavior of the resident 10 rising from the bed 50 by pre-storing information about the position and height of the bed 50. In addition, the apparatus 1 for detecting fall and rise may prevent an accident from occurring to a patient with an advanced disease by detecting whether or not the resident 10 has left the bed 50.

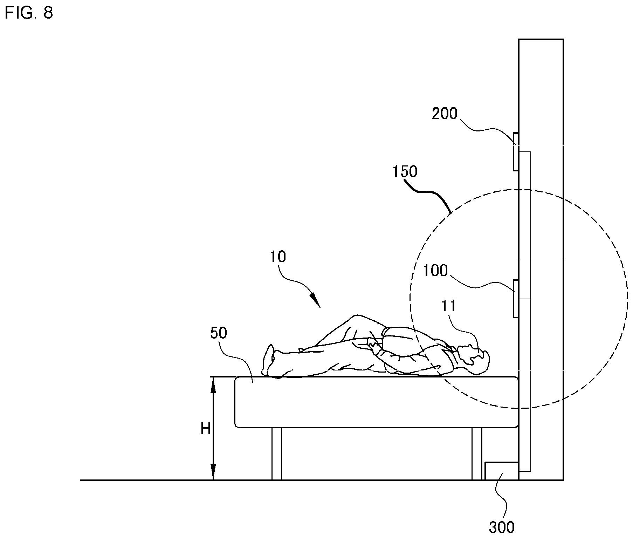

FIG. 8 is a view illustrating sensing as to whether or not a resident is lying on a bed according to one embodiment of the present invention.

Referring to FIGs. 2 and 8, the first sensor unit 100 may be disposed on the bed 50 on which the resident 10 is located. The first sensor unit 100 may have a sensing region 150 to determine whether or not the resident 10 has left the bed 50. The sensing region 150 of the first sensor unit 100 may be a region in which the first sensor unit 100 may sense a biometric signal of the resident 10 lying on the bed 50. The first sensor unit 100 may sense a biometric signal generated from the specific position 11 by sensing the specific position 11 of the resident 10 located within the sensing region 150.

When the resident 10 is present within the sensing region 150, the controller 300 may determine that the resident 10 is not in danger. When the resident 10 is present within the sensing region 150, the communication unit 500 may not generate an alarm.

For example, if the height H of the bed 50 is low, a biometric signal from the resident 10 lying on the bed 50 may be sensed around the floor of the indoor space. When the biometric signal from the resident 10 lying on the bed 50 is sensed around the floor of the indoor space, the controller 300 may erroneously recognize that the resident 10 has fallen down on the floor even though the resident 10 is lying on the bed 50. Therefore, when the resident 10 is located within the sensing region 150 of the first sensor unit 100, the controller 300 may determine that the resident 10 has not fallen but is lying on the bed 50. For example, the sensing region 150 may not extend to the floor of the indoor space, and may extend only to the upper surface of the bed 50 having a low height. That is, the sensing region 150 may be determined in consideration of the height of the bed 50 which is pre-stored.

As is apparent from the above description, an apparatus for detecting fall and rise according to one embodiment of the present invention senses whether or not a falling behavior and a rising behavior of a resident occur, and may thus increase reliability of determination of the falling behavior of the resident. The apparatus for detecting fall and rise determines the falling behavior of the resident based on whether or not the resident rises within a designated time after the falling behavior of the resident, and may thus more accurately determine whether or not the resident has fallen due to a health problem.

The apparatus for detecting fall and rise according to one embodiment of the present invention sets a first sensor unit and a second sensor unit such that, among three axis coordinates of the first sensor unit and the second sensor unit, two axis coordinates thereof coincide with each other, and may thus sense the rising and falling behaviors of the resident using only information about shortest distances from the first sensor unit and the second sensor unit to the resident and information about a distance between the first sensor unit and the second sensor unit.

When the apparatus for detecting fall and rise according to one embodiment of the present invention determines that the resident has fallen, the apparatus for detecting fall and rise automatically outputs a warning message, and thus, persons receiving the warning message may prepare for occurrence of a secondary accident involving the resident having a health problem.

The apparatus for detecting fall and rise according to one embodiment of the present invention may sense a specific position of the resident so as to determine a change in the height of the resident, and change sensing regions of the sensing units from the existing specific position to a new specific position so as to sense a biometric signal after the resident has fallen. Thereby, the apparatus for detecting fall and rise may increase reliability in sensing of the biometric signal while sensing a change in the height of the resident.

The invention has been described in detail with reference to preferred embodiments thereof. However, it will be appreciated by those skilled in the art that changes may be made in these embodiments without departing from the principles and spirit of the invention, the scope of which is defined in the appended claims and their equivalents

## Claims

1. An apparatus for detecting fall and rise, comprising:

   a first sensor unit (100) disposed at a first height and configured to sense a movement of a resident (10);
   a second sensor unit (200) disposed at a second height higher than the first height and configured to sense the movement of the resident (10); and
   a controller (300) configured to sense a falling behavior and a rising behavior of the resident (10) based on distances from the resident (10) measured by the first sensor unit (100) and the second sensor unit (200),
   wherein the controller (300) sets the first sensor unit (100) and the second sensor unit (200) such that, among three axis coordinates of the first sensor unit (100) and the second sensor unit (200) in a 3D orthogonal coordinate system, two axis coordinates thereof coincide with each other.

2. The apparatus (1) of claim 1, wherein the controller (300) calculates a height (z2) of the resident (10) based on the distances (R1, R2) from the resident (10) measured by the first sensor unit (100) and the second sensor unit (200), and determines the falling behavior and the rising behavior of the resident (10) using the height (z2) of the resident (10).

3. The apparatus (1) of claim 2, wherein:

   the first sensor unit (100) and the second sensor unit (200) measure respective shortest distances (R1, R2) from the resident (10); and
   the controller (300) calculates the height (z2) of the resident (10) based on the shortest distances (R1, R2), the coordinates of the first sensor unit (100), and the coordinates of the second sensor unit (100).

4. The apparatus (1) of claim 1, wherein, when the rising behavior of the resident (10) is not sensed within a designated time after sensing the falling behavior of the resident (10), the controller (300) determines that the resident (10) is in danger.

5. The apparatus (1) of claim 4, wherein:

   the first sensor unit (100) and the second sensor unit (200) sense biometric signals of the resident (10) at specific position (11)s of the resident (10); and
   the specific position (11) means a position of a body part of the resident (10) at which a shortest distance (R1, R2) from each of the first sensor unit (100) and the second sensor unit (200) to the resident (10) is derived.

6. The apparatus (1) of claim 1, wherein, when specific position (11)s of the resident (10) sensed by the first sensor unit (100) and the second sensor unit (200) are different, the controller (300) calculates a height (z2) of the resident (10) assuming that a first specific position (11) sensed by the first sensor unit (100) and a second specific position (11) sensed by the second sensor unit (200) are the same.

7. The apparatus (1) of claim 1, wherein, when a biometric signal is not sensed from a specific position (11) of the resident (10) sensed by each of the first sensor unit (100) and the second sensor unit (200), the controller (300) controls each of the first sensor unit (100) and the second sensor unit (200) so as to change the specific position (11) of the resident (10) sensed by each of the first sensor unit (100) and the second sensor unit (100).

8. The apparatus (1) of claim 7, wherein, when a biometric signal is sensed from the changed specific position (11), the controller (300) determines the falling behavior and the rising behavior by calculating a height (z2) of the resident (10) based on the changed specific position (11).

9. The apparatus (1) of claim 1, wherein, when a bed (50) is disposed in a residence place of the resident (10), the controller (300) pre-stores information about a distance (D1) between an axis on which the first sensor unit (100) and the second sensor unit (200) are disposed, the bed (50) and a height (H) of the bed (50).

10. The apparatus (1) of claim 9, wherein the controller (300) does not determine a change in the movement of the resident (10) within a designated range from the distance (D1) between the axis and the bed (50) as the falling behavior based on the information about the distance (D1) between the axis and the bed (50).

11. The apparatus (1) of claim 9, wherein, when a position of the resident (10) sensed by the first sensor unit (100) and the second sensor unit (200) is within an error range from the height (H) of the bed (50), the controller (300) determines that the resident (10) is located on the bed (50) and is thus not in danger.

12. The apparatus (1) of claim 9, wherein:

the controller (300) sets a rising height of the resident (10) based on the height (H) of the bed (50); and
the controller (300) determines that the resident (10) has risen when a position of the resident (10) is higher than the rising height.

13. The apparatus (1) of claim 9, wherein the controller (300) determines whether or not a position of the resident (10) deviates from the bed (50) beyond an error range using the height (H) of the bed (50) and the distance (D1) between the axis and the bed (50).

14. The apparatus (1) of claim 1, wherein:

the first sensor unit (100) is disposed on a bed (50), on which the resident (10) is located;
the first sensor unit (100) has a sensing region (150) to determine whether or not the resident (10) has left the bed (50); and
the controller (300) determines that the resident (10) is not in danger, when the resident (10) is present within the sensing region (150).

15. The apparatus (1) of claim 1, further comprising a database (400) configured to store behavior pattern information about human movements, wherein the controller (300) determines a state of the resident (10) by comparing a pattern of the movement of the resident (10) sensed by the first sensor unit (100) and the second sensor unit (200) with the behavior pattern information stored in the database (400).

FIG. 1

FIG. 2

FIG. 3

FIG. 4

FIG. 5a

FIG. 5b

FIG. 6

FIG. 7

FIG. 8